# EUROPEAN PATENT SPECIFICATION

(11) **EP 2 906 345 B1**
(45) Date of publication and mention of the grant of the patent: **04.12.2019**
(21) Application number: 13803243.8
(22) Date of filing: 08.10.2013
(51) Int. Cl.: B01J 37/03, B01J 37/10, B01J 21/06, B01J 23/89, B01J 37/08, C07C 5/11, C07C 13/20

(54) **RUTHENIUM-BASED CATALYST AND USE THEREOF IN THE SELECTIVE HYDROGENATION OF AROMATIC OR POLYUNSATURATED COMPOUNDS**
RUTHENBASIERTER KATALYSATOR UND VERWENDUNG DAVON BEI DER SELEKTIVEN HYDRIERUNG AROMATISCHER ODER MEHRFACH UNGESÄTTIGTER VERBINDUNGEN
CATALYSEUR À BASE DE RUTHÉNIUM ET SON UTILISATION DANS L'HYDROGÉNATION SÉLECTIVE DE COMPOSÉS AROMATIQUES OU POLYINSATURÉS

(30) Priority: 12.10.2012 IT MI20121724
(43) Date of publication of application: 19.08.2015
(73) Proprietor: Versalis S.p.A., 20097 San Donato Milanese (MI) (IT)
(72) Inventor: MASI, Francesco, I-26866 Sant'angelo Lodigiano (LO) (IT); SOMMAZZI, Anna, I-16038 Santa Margherita Ligure (GE) (IT); BENCINI, Elena, I-46030 Virgilio (MN) (IT); RONCHIN, Lucio, I-31021 Mogliano Veneto (TV) (IT); TONIOLO, Luigi, I-35031 Abano Terme (PD) (IT); VAVASORI, Andrea, I-30133 Venezia (VE) (IT)
(74) Representative: Bottero, Carlo
(86) International application number: PCT/IB2013/059195
(87) International publication number: WO 2014/057417

(56) References cited:
- EP-A1- 0 554 765
- EP-A1- 2 446 963
- JP-A- H08 253 433
- US-A- 4 734 536
- US-A- 5 157 179

## Description

The present invention relates to a new catalyst based on ruthenium and its use in the selective hydrogenation of aromatic or polyunsaturated compounds.

More specifically, the present invention relates to a new catalyst based on ruthenium and its use in the selective hydrogenation of aromatic hydrocarbons, for example benzene and/or toluene and xylenes or polyunsaturated compounds such as dienes or α,β-unsaturated carbonyls.

The selective hydrogenation reaction of benzene to cyclohexene, for example, has become part of the range of reactions of interest in the industrial chemistry due to the importance of this olefin as an intermediate product of numerous processes relating to the production of polymeric materials.

As is known, the selective hydrogenation reaction of benzene to cyclohexene is a heterogeneous gas/liquid/solid reaction which takes place in the presence of a heterogeneous catalyst essentially consisting of ruthenium supported on an inert solid, insoluble in the reaction medium. The liquid phase is, in turn, composed of two phases, an organic phase and an aqueous phase, the latter favouring the desorption of cyclohexene from the surface of the catalyst.

In scientific literature, there are numerous documents relating to the preparation of catalytic systems based on ruthenium, suitable for the selective hydrogenation of polyunsaturated compounds. Mention can be made, for example, of USA patents US 4,734,536 and US 5,589,600 which refer to ruthenium catalysts supported on zirconia and methods for their preparation.

MI2010A001988 (EP2446963) describes a new catalyst based on ruthenium comprising a zirconia carrier, iron oxide as promoter and, as a further promoter, at least one oxide of a metal selected from those of groups IB, IIB and IIIA.

The Applicant has now found new catalysts based on ruthenium containing additional promoters based on Ti, V, Zr and/or Hf which are particularly active in selective hydrogenation reactions.

An object of the present invention therefore relates to a catalyst suitable for the selective hydrogenation of aromatic or polyunsaturated compound, comprising:
- a zirconia carrier (ZrO₂)
- ruthenium
- iron oxide (Fe₂O₃)
- at least one promoter (A) selected from the oxides of metals of groups IB, IIB and IIIA
- at least one promoter (B) containing V, Ti, Hf, Zr or a mixture thereof, obtained by hydrolysis of at least one precursor P-B selected from the following metal arenes:

   V°(arene)₂ (I)

   V(arene)₂ AlCl₄ (II)

   M(η⁶-arene)ₚAl_{q}Xᵣ (III)

   M(η⁶-arene)_{p'}Al_{q'}X_{r'}Rₛ (IV)

   wherein:
   - V represents vanadium,
   - Al represents aluminium,
   - M represents zirconium (Zr), hafnium (Hf), titanium (Ti) or mixtures thereof, preferably titanium or zirconium;
   - arene represents a benzene, or a benzene substituted by from 1 to 6 linear or branched C₁-C₆ alkyl groups, or mixtures thereof;
   - X represents a halogen atom, selected from chlorine, bromine, fluorine, iodine, preferably chlorine;
   - R represents a linear or branched C₁-C₁₅ alkyl group;
   - p represents a number ranging from 1 to 2;
   - q is a number ranging from 2 to 6, preferably from 2 to 3;
   - r is a number ranging from 8 to 20, preferably from 8 to 11;
   - p' represents a number ranging from 1 to 2;
   - q' is a number ranging from 2 to 6, preferably from 2 to 3;
   - r' is a number ranging from 2 to 20, preferably from 6 to 9;
   - s is a number ranging from 1 to 6, preferably 2.

For the purposes of the present description and following claims, the definitions of the numerical ranges always include the extremes, unless otherwise specified.

The ruthenium content in the catalytic system, expressed as metal element, ranges from 20 to 55% by weight, with respect to the weight of the final catalyst. In the final catalyst, the ruthenium is in the form of metallic ruthenium.

The iron oxide ranges from 1 to 20% by weight with respect to the weight of the final catalyst.

The promoter (A) is preferably selected from gallium oxide (Ga₂O₃), zinc oxide (ZnO), copper oxide (CuO) and mixtures thereof. Even more preferably, a mixture of gallium oxide and zinc oxide is used, or a mixture of copper oxide and zinc oxide; a mixture of gallium oxide and zinc oxide is particularly preferred. The promoters (A) are present in quantities ranging from 1 to 20% by weight with respect to the ruthenium metal. The molar ratio between the promoter (A) and each of the other promoters (A) present in the catalyst, expressed as molar ratio between the oxides, ranges from 0.5 to 1.5, preferably from 0.9 to 1.1.

The promoter(s) (B) containing Ti, Zr, Hf and/or V, derive from one or more of the precursors P-B selected from the metal arenes mentioned above having formula (I), (II), (III) and (IV), and are generated from these during the preparation of the catalyst, in particular by hydrolysis. Said hydrolysis, as described in greater detail hereunder, takes place during the preparation stage subsequent to the precipitation of the iron oxides and metals of groups IB, IIB and IIIA.

The metals Ti, Zr, Hf and V are present in the final catalyst in quantities ranging from 0.2 to 5% by weight, expressed as the weight of the metal with respect to the weight of the catalyst.

When more than one metal selected from Ti, Zr, Hf and V is present, the molar ratio between one of said metals and each of the other metals Ti, Zr, Hf and V present in the catalysts, ranges from 0.5 to 2.

The vanadium arenes having general formula (I) and (II) and their preparation are described, for example, in patents EP0398402, EP0437897, EP0525857.

The compounds having formula (IV) and their preparation are described, for example, in EP 684263, in MI2010A002400, in MI2010A002401 and in "The crystal structure of (η6-C6Me6)Ti(µ-Cl)2·(AlClEt]2 and the catalytic activity of the (C6Me6)TiAl2Cl8Etx(X=0-4) complexes towards butadiene" in Journal of Organometallic Chemistry (1992), Vol. 430, 317-325.

The compounds having formula (III) and their preparation are also described in: Biagini et al. Journal of Organometallic Chemistry 1988, 355, 99; in Calderazzo et al. Journal of the Chemical Society. Dalton Transactions. (1990) pages 1813-1817, in MI2010A002400 and in MI2010A002401.

The compounds having general formula (III) can also be obtained according to processes known in the art, as described, for example, by Troyanov et al. in "Synthesis of arene Ti and Zr complexes and their reactivity towards air: crystal structure of [(C6H3Me3)2Zr(AlCl4)](Al2Cl7) and TiCl3(OPh)". Journal of Organometallic Chemistry (1995), Vol. 494, C4-C7; or in "The synthesis and crystal structure of the n-benzenezirconium(III) bromoaluminate complex {(µ2-Br)3[(η6-C6H6)Zr(µ2-Br)2·AlBr2]2}(Al2Br7)·2.5C6H6 and the n-benzene-zirconium(II) iodoaluminate complex [(η-C6H6)2Zr(µ2-I)2AlI2](Al3I10)·0.5C6H6", Organometallic Chemistry in the USSR (1989), Vol. 2(6), pages 732-736; or in "The synthesis and crystal structure of the n-mesitylenezirconium(II) bromide complexes [(η6-C6H3Me3)2Zr(µ-Br)2AlBr2]·(Al2Br7) and [(η6-C6H3Me3)2Zr(µ-Br)2·AlBr2](Al3OBr8)", Organometallic Chemistry in the USSR (1992), Vol. 5(5), pages 527-530; "Arene Complexes of Titanium and Zirconium in Low Oxidation States: Crystal Structures of β-(η6C6H6)Ti(AlI4)2, [η6-(C6Me6)3Zr3Br6](Al2OBr8)(Al2Br7)·(C6H6), [η6-C6H3Me3)3Zr3Br6] (Al3OBr8)3, and [(η6C6H6)2Zr(AlBr4)](Al2Br7)·2(C6H6)," Russian Journal of Coordination Chemistry (1997), Vol. 23, No. 12, pages 836-843; "Syntheses and Crystal Structures of [(η6-Diarene) Ti-bis(tetrachloroaluminate) Complexes, Diarene = Biphenyl or 3,5,3',5'-Tetramethyl-biphenyl", Z. Anorg. Allg. Chem. (2001), vol. 627, 1423-1425; "Arene complexes of titanium and zirconium. Synthesis and structure" in Journal of Organometallic Chemistry (1994), Vol. 475, 139-147: (η⁶-arene) Ti(AlCl₄)₂, {[η⁶-[(C₆H₆)₃]Zr(µ-Br₂)AlBr₂]₂}(Al₂Br₇) ; "Preparation of Halogen-modified Ti(II) Arene Complexes and their Electronic Spectra" in Transition Met. Chem. (1978), vol. 3, 127-130; "AreneTitanium(II) Complexes of formula Ar·TiCl2·Al2Cl6 in Journal of Organometallic Chemistry (1975), Vol. 87, 295-299; "The synthesis of Titanium(II) complexes containing methylbenzene ligands" in Journal of Organometallic Chemistry (1973), Vol. 54, 203-205.

The ruthenium-based catalyst, object of the present invention, can be prepared by precipitating, in various steps and in a certain succession, the hydrolyzable salts of the single metals and adding one or more precursors P-B selected from those listed above.

In particular, the preparation process of the catalysts of the present invention comprises:
- a precipitation phase in a basic aqueous solution of a zirconium alcoholate which precipitates in the form of finely subdivided ZrO₂,
- a precipitation phase of the iron oxide and promoter oxides (A) by the addition of the corresponding hydrolyzable salts,
- an addition phase of one or more precursors P-B of the promoter(s) (B), wherein the precursor of the promoter (B) is selected from metal arenes having general formula (I), (II), (III), (IV) or mixtures thereof, indicated above,
- an addition phase of a hydrolyzable salt of ruthenium,
- hydrothermal treatment of the suspension resulting from the previous phases.

According to a preferred embodiment of the present invention, in the metal arenes having general formula (I), (II), (III) or (IV), said arenes can be preferably selected from: benzene, toluene, ortho-xylene, meta-xylene, para-xylene, 1,2,3-trimethylbenzene, 1,2,4-trimethyl benzene, 1,3,5-trimethylbenzene (mesitylene), hexamethylbenzene, or mixtures thereof. Benzene, toluene, 1,3,5-trimethylbenzene (mesitylene) are preferred.

Specific examples of the metal arene having general formula (III), used in the present invention, are, for example:
- Zr(η⁶-benzene)₂Al₃Cl₁₁;
- Zr(η⁶-benzene)₂Al₃Br₁₁;
- Zr(η⁶-mesitylene)₂Al₃Br₁₁;
- Zr(η⁶-mesitylene)₂Al₃Cl₁₁;
- Zr(η⁶-benzene)Al₂Cl₈;
- Zr(η⁶-toluene)Al₂Cl₈;
- Zr(η⁶-mesitylene)Al₂Cl₈;
- Zr(η⁶-benzene)Al₂Br₈;
- Zr(η⁶-toluene)Al₂Br₈;
- Zr(η⁶-mesitylene)Al₂Br₈;
- Ti(η⁶-benzene)Al₂Cl₈;
- Ti(η⁶-toluene)Al₂Cl₈;
- Ti(η⁶-xylene)Al₂Cl₈;
- Ti(η⁶-mesitylene)Al₂Cl₈;
- Ti(η⁶-benzene)Al₂Br₈;
- Ti(η⁶-toluene)Al₂Br₈;
- Ti(η⁶-mesitylene)Al₂Br₈.

According to a preferred embodiment of the present invention, in the metal alkyl-arene having general formula (IV) said group R can preferably be selected from: ethyl, n-propyl, iso-propyl, n-butyl, sec-butyl, tert-butyl, iso-butyl, n-pentyl, n-hexyl, n-heptyl, n-octyl, n-decyl, n-tridecyl. Ethyl, n-butyl, sec-butyl, iso-butyl, n-pentyl n-octyl, n-tridecyl are preferred.

Specific examples of the metal alkyl-arene having general formula (IV), used in the present invention, are, for example:

Zr(η⁶-benzene)₂Al₃Cl₉(n-butyl)₂;

- Zr(η⁶-benzene)₂Al₃Cl₉(n-octyl)₂;
- Zr(η⁶-mesitylene)₂Al₃Cl₉(butyl)₂;
- Zr(η⁶-mesitylene)₂Al₃Cl₉(n-octyl)₂;
- Zr(η⁶-toluene)₂Al₃Cl₉(butyl)₂;
- Zr(η⁶-toluene)₂Al₃Cl₉(n-octyl)₂;
- Zr(η⁶-benzene)Al₂Cl₆(n-octyl)₂;
- Zr(η⁶-benzene)Al₂Cl₆(n-octyl)₂;
- Zr(η⁶-benzene)Al₂Cl₃(n-octyl)₅;
- Zr(η⁶-benzene)Al₂Cl₅(n-octyl)₃;
- Zr(η⁶-benzene)Al₂Cl₄(n-octyl)₄;
- Zr(η⁶-toluene)Al₂Cl₆(n-octyl)₂;
- Zr(η⁶-toluene)Al₂Cl₃(n-octyl)₅;
- Zr(η⁶-toluene)Al₂Cl₅(n-octyl)₃;
- Zr(η⁶-toluene)Al₂Cl₄(n-octyl)₄;
- Zr(η⁶-mesitylene)Al₂Cl₆(n-octyl)₂;
- Zr(η⁶-mesitylene)Al₂Cl₃(n-octyl)₅;
- Zr(η⁶-mesitylene)Al₂Cl₅(n-octyl)₃;
- Zr(η⁶-mesitylene)Al₂Cl₄(n-octyl)₄;
- Zr(η⁶-benzene)Al₂Cl₆(n-butyl)₂;
- Zr(η⁶-benzene)Al₂Cl₆(iso-butyl)₂;
- Zr(η⁶-benzene)Al₂Cl₃(n-butyl)₅;
- Zr(η⁶-benzene)Al₂Cl₅(n-butyl)₃;
- Zr(η⁶-benzene)Al₂Cl₄(n-butyl)₄;
- Zr(η⁶-benzene)Al₂Cl₄(etile)₄;
- Zr(η⁶-toluene)Al₂Cl₆(n-butyl)₂;
- Zr(η⁶-toluene)Al₂Cl₃(n-butyl)₅;
- Zr(η⁶-toluene)Al₂Cl₅(n-butyl)₃;
- Zr(η⁶-toluene)Al₂Cl₄(n-butyl)₄;
- Zr(η⁶-mesitylene)Al₂Cl₆(n-butyl)₂;
- Zr(η⁶-mesitylene)Al₂Cl₆(sec-butyl)₂;
- Zr(η⁶-mesitylene)Al₂Cl₆(iso-butyl)₂;
- Zr(η⁶-mesitylene)Al₂Cl₆(n-pentyl)₂;
- Zr(η⁶-mesitylene)Al₂Cl₆(ethyl)₂;
- Zr(η⁶-mesitylene)Al₂Cl₃(n-butyl)₅;
- Zr(η⁶-mesitylene)Al₂Cl₅(n-butyl)₃;
- Zr(η⁶-mesitylene)Al₂Cl₄(n-butyl)₄;
- Ti(η⁶-benzene)Al₂Cl₆(n-octyl)₂;
- Ti(η⁶-benzene)Al₂Cl₆(n-octyl)₂;
- Zr(η⁶-benzene)Al₂Cl₃(n-octyl)₅;
- Ti(η⁶-benzene)Al₂Cl₅(n-octyl)₃;
- Ti(η⁶-benzene)Al₂Cl₄(n-octyl)₄;
- Ti(η⁶-toluene)Al₂Cl₆(n-octyl)₂;
- Ti(η⁶-toluene)Al₂Cl₃(n-octyl)₅;
- Ti(η⁶-toluene)Al₂Cl₅(n-octyl)₃;
- Ti(η⁶-toluene)Al₂Cl₄(n-octyl)₄;
- Ti(η⁶-mesitylene)Al₂Cl₆(n-octyl)₂;
- Ti(η⁶-mesitylene)Al₂Cl₃(n-octyl)₅;
- Ti(η⁶-mesitylene)Al₂Cl₅(n-octyl)₃;
- Ti(η⁶-mesitylene)Al₂Cl₄(n-octyl)₄;
- Ti(η⁶-benzene)Al₂Cl₆(n-butyl)₂;
- Ti(η⁶-benzene)Al₂Cl₆(iso-butyl)₂;
- Ti(η⁶-benzene)Al₂Cl₃(n-butyl)₅;
- Ti(η⁶-benzene)Al₂Cl₅(n-butyl)₃;
- Ti(η⁶-benzene)Al₂Cl₄(n-butyl)₄;
- Ti(η⁶-benzene)Al₂Cl₄(ethyl)₄;
- Ti(η⁶-toluene)Al₂Cl₆(n-butyl)₂;
- Ti(η⁶-toluene)Al₂Cl₃(n-butyl)₅;
- Ti(η⁶-toluene)Al₂Cl₅(n-butyl)₃;
- Ti(η⁶-toluene)Al₂Cl₄(n-butyl)₄;
- Ti(η⁶-mesitylene)Al₂Cl₆(n-butyl)₂;
- Ti(η⁶-mesitylene)Al₂Cl₆(sec-butyl)₂;
- Ti(η⁶-mesitylene)Al₂Cl₆(iso-butyl)₂;
- Ti(η⁶-mesitylene)Al₂Cl₆(n-pentyl)₂;
- Ti(η⁶-mesitylene)Al₂Cl₆(ethyl)₂;
- Ti(η⁶-mesitylene)Al₂Cl₃(n-butyl)₅;
- Ti(η⁶-mesitylene)Al₂Cl₅(n-butyl)₃;
- Ti(η⁶-mesitylene)Al₂Cl₄(n-butyl)₄.
In particular the following steps are effected:
(a) a zirconium alcoholate in an alcohol solution, preferably a solution of the alcohol corresponding to the alcoholate, is added to a basic aqueous solution, preferably a KOH solution, obtaining the formation of a precipitate of ZrO₂;
(b) a hydrolyzable iron salt is added and at least one hydrolyzable salt of a metal selected from metals of groups IB, IIB and IIIA;
(c) the precipitate is separated and dried;
(d) the solid resulting from the drying is resuspended in a solvent selected from benzene, toluene and xylenes and one or more P-B precursors of promoters (B), selected from metal-arenes having formula (I), (II), (III) or (IV) in solution of the same solvent, are added to the resulting suspension;
(e) the solid resulting from the previous step is separated, re-dispersed in an aqueous solution of a base, preferably KOH, and a hydrolyzable salt of ruthenium is added, obtaining the precipitation of a solid;
(f) the solid obtained in the previous step is separated, re-dispersed in water, and is kept in an autoclave under hydrothermal conditions, preferably at a temperature ranging from 120 to 180°C and a hydrogen pressure ranging from 3 to 6 MPa;
(g) the solid obtained in step (f) is separated.

In step (a), a preferred aspect is to use Zr(OPr)₄ in isopropanol. This step is effected under stirring, and the precipitate of ZrO₂ which is formed is finely subdivided.

The hydrolyzable salts which are added to this suspension precipitate in the form of oxides, therefore generating iron oxide and the promoters (A). Hydrolyzable salts which can be suitably used are all hydrolyzable salts of iron and metals of groups IB, IIB, IIIA. Salts of Fe, Ga, Cu, and Zn are preferably used and in particular the relative chlorides and sulfates. FeCl₃ heptahydrate, Ga₂ (SO₄)₃ hydrate, ZnSO₄ heptahydrate, ZnCl₂, CuCl₂, CuSO₄ pentahydrate can be used, for example.

The solid thus obtained is separated from the aqueous phase, for example by centrifugation, and dried, for example in an oven, at a temperature ranging from 90 to 110°C.

Benzene is preferably used in step (d) as solvent.

The suspension obtained in step (d) is preferably left under stirring at room temperature for a time ranging from 12 to 48 hours, water is then added and the solid is recovered by centrifugation, washed, for example with ethyl ether, and dried in an oven at a temperature ranging from 95 to 150°C.

In step (e) the hydrolyzable salt of Ru can be selected from chloride and nitrosyl nitrate and, for example, is RuCl₃. The base, in particular potash, is used in a concentration ranging from 5 to 20%, for example 10% by weight. The suspension is heated to 80°C then left to digest for a period ranging from 12 to 48 hours, for example 20 hours.

Without adhering to any theory, it is believed that during step (d), the adsorption takes place of the precursors P-B in the carrier obtained in steps a-c and a first decomposition starts, of the redox type, of the precursors P-B. It is then deemed that in step (e), the complete hydrolysis takes place of said precursors P-B on the part of the aqueous solution of potash. The subsequent precipitation of the Ru precursor on the carrier thus obtained is then decisive for the final reactivity of the catalyst.

In steps (f) and (g), the solid obtained is separated, for example, by centrifugation. At the end of step (f) the suspension is discharged from the autoclave and left under stirring in order to obtain the passivation of Ru. The solid is then isolated and dried.

The active phase of the catalyst is obtained by reduction with hydrogen, preferably operating at a temperature ranging from 120 to 180°C and a pressure ranging from 20 to 80 bar.

An object of the present invention also relates to the catalysts obtained with the preparation method described above.

The precursors P-B of the promoters (B) having formula (III) or (IV) can be prepared according to MI2010A002400, also extending to Ti what has been described for Hf and Zr: in particular the compounds having formula (IV) are obtained by operating in the presence of an alkylating agent, whereas for the preparation of the compounds having formula (III), the same type of process can be used, but without adopting any alkylating agent. Said preparation process of the precursors of formula (III) and (IV) comprises reacting the following components:
(i) at least one metal halide having general formula (a) :

   MX₄ (a)

   wherein:
   - M represents zirconium (Zr), titanium (Ti), hafnium (Hf) or mixtures thereof, zirconium is preferred;
   - X represents a halogen atom selected from chlorine, bromine, fluorine and iodine, preferably chlorine;
(ii) aluminium metal;
(iii) optionally at least one aluminium halide having general formula (b):

   Al X₃ (b)

   wherein X represents a halogen atom selected from chlorine, bromine and iodine, preferably chlorine;
(iv) at least one arene;
(v) possibly at least one alkylating agent selected from:
   - metal alkyls having general formula (c):

      M₁(R)ₘ (c)

      wherein M₁ represents aluminium, magnesium, zinc, lithium; R represents a linear or branched C₁-C₁₅ alkyl group; m is 1, 2 or 3;
   - aluminium alkyl chlorides having general formula (d):

      Al(R)ₙCl₃₋ₙ (d)

      wherein R represents a linear or branched C₁-C₁₅ alkyl group; n is 1 or 2.

Said arene is preferably selected from: benzene, toluene, ortho-xylene, meta-xylene, para-xylene, 1,2,3-trimethylbenzene, 1,2,4-trimethylbenzene, 1,3,5-trimethylbenzene (mesitylene), hexamethylbenzene, or mixtures thereof. Benzene, toluene, 1,3,5-trimethylbenzene (mesitylene) or mixtures thereof are preferred.

Said metal alkyls, when used, may preferably be selected from lithium n-butyl, lithium sec-butyl, lithium tert-butyl, lithium iso-butyl, lithium n-pentyl, aluminium tri-ethyl, aluminium tri-iso-butyl, aluminium tri-octyl, butyl-octyl-magnesium, or mixtures thereof.

Said aluminium alkyl chlorides, when used, may preferably be selected from: di-ethyl-aluminium chloride, mono-ethyl-aluminium dichloride, n-octyl-aluminium dichloride, di-methyl-aluminium chloride, di-iso-butyl-aluminium chloride, iso-butyl-aluminium dichloride, ethyl-aluminium sesquichloride, or mixtures thereof.

Said preparation process of the precursor of the promoter (B) can be carried out operating at a molar ratio between the aluminium present in the aluminium halide having general formula (b) and the metal present in the metal halide having general formula (a), ranging from 1 to 4, preferably from 1.5 to 3.

According to a further embodiment, the preparation process in particular of the precursor having formula (IV), can be carried out operating at a molar ratio between the metal present in the metal alkyls having general formula (c) and the metal present in the metal halide having general formula (a), ranging from 0.5 to 10, preferably between 1 and 2.

According to a further embodiment, said preparation process of the precursor having formula (IV) can be carried out operating at a molar ratio between the aluminium present in the aluminium alkyl chlorides having general formula (d) and the metal present in the metal halide having general formula (a), ranging from 1 to 6, preferably from 2 to 3.

According to an embodiment, said preparation process of the precursor of the promoter (B) can be carried out in the presence of an organic solvent, preferably an aliphatic or aromatic hydrocarbon solvent such as, for example, n-pentane, n-hexane, blends of hexanes, n-heptane, n-octane, n-nonane, n-decane, n-undecane, n-dodecane, benzene, toluene, xylene, 1,3,5-trimethylbenzene (mesitylene).

According to an embodiment, said preparation process of the precursor (III) or (IV) can be carried out at a temperature ranging from room temperature (20-25°C) to the reflux temperature of the suspension obtained by putting the above components (i), (ii,) (iv), optionally (v) and (iii) in contact, preferably at the reflux temperature of the suspension obtained by putting the above components in contact.

According to an embodiment, said preparation process of the precursor (III) or (IV) can be carried out for a period of time ranging from 1 hour to 25 hours, preferably from 3 to 24 hours. At the end of the above process, the precipitation of the alkyl arene metal (IV) or arene metal (III) can be obtained in the form of a solid, for example by the addition of an organic solvent such as, for example, n-pentane, n-hexane, n-heptane; or a solution comprising said alkyl arene metal (iv), or said arene metal (III) or an oil comprising said alkyl arene metal (IV) or said arene metal (III), can be obtained. Said solution and said oil can be filtered in order to eliminate the aluminium metal in excess.

Alternatively, the alkyl arene metal having general formula (IV) can be obtained, according to MI2010A002400 or MI2010A002401, through a process which comprises reacting a metal arene with at least one alkylating agent, extending also to Ti what has been described for Hf and Zr. Said process for the preparation of a metal alkyl arene having general formula (IV) comprises putting the following components in contact:
(i') at least one metal arene having general formula (III) :

   M(η⁶-arene)ₚ Al_{q}Xᵣ (III)

   wherein:
   - M represents zirconium (Zr), titanium (Ti), hafnium (Hf), or mixtures thereof, preferably zirconium;
   - arene represents a benzene, or a benzene substituted with 1 or 6 linear or branched C₁-C₆ alkyl groups or mixtures thereof;
   - X represents a halogen atom selected from chlorine, bromine, fluorine and iodine, preferably chlorine;
   - q is a number ranging from 2 to 6, preferably 3 when p is equal to 2, and preferably 2 when p is equal to 1;
   - r is a number ranging from 8 to 20, preferably 11 when p is equal to 2, 8 when p is equal to 1;
   - p is a number between 1 and 2
(ii') at least one alkylating agent selected from:
   - metal alkyls having general formula (c):

      M(R)ₘ (c)

      wherein M represents aluminium, magnesium, zinc, lithium; R represents a linear or branched C₁-C₁₅ alkyl group; m is 1, 2 or 3;
   - aluminium alkyl chlorides having general formula (d):

      Al(R)ₙCl₃₋ₙ (d)

      wherein R represents a linear or branched C₁-C₁₅ alkyl group; n is 1 or 2.

The preparations of the arenes having formula (III) are indicated in the references described in the previous pages, or said compounds having formula (III) can be prepared according to MI2010A002400, in the absence of the alkylating agent. It is possible to operate, for example, by putting the following components in contact, under reaction conditions,: aluminium metal, aluminium trichloride, metal tetrachloride and the preselected arene. Said components can be reacted at a temperature ranging from room temperature (20°C-25°C) to the reflux temperature of the reaction mixture obtained by putting said components in contact. At the end of the reaction, a suspension can be obtained comprising said metal arene, or a biphasic system (reaction raw product) which can be filtered in order to eliminate the unaltered aluminium metal in excess, obtaining a solution from which said metal arene in solid form is separated, for example, by precipitation in a hydrocarbon solvent, preferably aliphatic (e.g. n-heptane), or it may not be filtered.

The above process for the preparation of a metal alkyl arene having general formula (IV) comprising putting components (i') and (ii') in contact, can be carried out operating at a molar ratio between the metal present in the metal alkyls having general formula (c) or the aluminium present in the aluminium chlorides having general formula (d) and the metal present in the metal arene having general formula (III), ranging from 1 to 20, preferably from 1.2 to 15.

Said metal alkyls (c) may preferably be selected from: lithium n-butyl, lithium sec-butyl, lithium tert-butyl, lithium iso-butyl, lithium n-pentyl, aluminium tri-ethyl, aluminium tri-iso-butyl, aluminium tri-octyl, butyl-octyl-magnesium, or mixtures thereof.

Said aluminium alkyl chlorides (d) may preferably be selected from: di-ethyl-aluminium chloride, mono-ethyl-aluminium dichloride, n-octyl-aluminium dichloride, di-methyl-aluminium chloride, di-iso-butyl-aluminium chloride, iso-butyl-aluminium dichloride, ethyl-aluminium sesquichloride, or mixtures thereof.

The above-mentioned preparation process of the precursor (IV) of the promoter (B), which comprises putting components (i') and (ii') in contact, can be carried out in the presence of an organic solvent, preferably an aliphatic or aromatic hydrocarbon solvent, selected from those described above, and under the same operative conditions (i.e. temperature, time) described above, for the process which comprises putting components (i), (ii), (iv), optionally (v) and (iii), in contact. At the end of this process, the precipitation of the metal alkyl arene can be obtained in the form of a solid (e.g. by means of an organic solvent such as, for example, n-pentane, n-hexane, n-heptane), or a solution comprising said metal alkyl arene or an oil comprising said metal alkyl arene.

The catalysts of the present invention can be conveniently used in the selective hydrogenation of aromatic or polyunsaturated compounds. The aromatic compounds which can be selectively hydrogenated using the catalyst object of the present invention are, for example, benzene, toluene, xylenes, phenol, bisphenyl, bisphenol A, naphthalene, anthracene.

Polyunsaturated compounds which can be selectively hydrogenated using the catalyst object of the present invention are dienes, such as 1,3-butadiene or isoprene, or α,β-unsaturated carbonyl compounds such as acrolein, methyl methacrylate, methyl acrylate, ethyl acrylate, butyl acrylate, 1,3-butene-2-one, acrylamide, acrylonitrile.

The catalyst, object of the present invention, may preferably be used in the selective hydrogenation of benzene to cyclohexene.

A further object of the present invention therefore relates to a process for the selective hydrogenation of aromatic or polyunsaturated compound which comprises putting aromatic or polyunsaturated compounds in contact with hydrogen in the presence of the catalyst of the present invention containing:
- a zirconia carrier (ZrO₂)
- ruthenium
- iron oxide (Fe₂O₃)
- at least one promoter (A) selected from the oxides of metals of groups IB, IIB and IIIA
- at least one promoter (B) containing V, Ti, Hf, Zr or a mixture thereof, obtained by hydrolysis of at least one precursor P-B selected from the following metal arenes:

   V°(arene)₂ (I)

   V(arene)₂ AlCl₄ (II)

   M(η⁶-arene)ₚAl_{q}Xᵣ (III)

   M(η⁶-arene)_{p'}Al_{q'}X_{r'}Rₛ (IV)

   wherein:
   - V represents vanadium,
   - Al represents aluminium,
   - M represents zirconium (Zr), hafnium (Hf), titanium (Ti) or mixtures thereof, preferably zirconium;
   - arene represents a benzene, or a benzene substituted by from 1 to 6 linear or branched C₁-C₆ alkyl groups, or mixtures thereof;
   - X represents a halogen atom, selected from chlorine, bromine, fluorine, iodine, preferably chlorine;
   - R represents a linear or branched C₁-C₁₅ alkyl group;
   - p represents a number ranging from 1 to 2;
   - q is a number ranging from 2 to 6, preferably from 2 to 3;
   - r is a number ranging from 8 to 20, preferably from 8 to 11;
   - p' represents a number ranging from 1 to 2;
   - q' is a number ranging from 2 to 6, preferably from 2 to 3;
   - r' is a number ranging from 2 to 20, preferably from 6 to 9;
   - s is a number ranging from 1 to 6, preferably 2.

In particular said process can include:
1. charging the heterogeneous catalyst of the present invention into an aqueous solution containing an alkaline hydroxide, said catalyst comprising a zirconia, ruthenium, iron oxide carrier, one or more promoter oxides (B) selected from the oxides of metals of group IB, IIB, IIIA and mixtures thereof, and one or more promoters (B) containing Ti, Zr, V or Hf deriving from the precursors P-B having formula (I), (II), (III) according to what has been previously indicated;
2. charging the aromatic or polyunsaturated compound into the aqueous solution, under stirring;
3. reacting the dispersed aromatic or polyunsaturated compound with a continuous flow of hydrogen;
4. at the end of the reaction, recovering the selectively hydrogenated compound by demixing the organic phase from the aqueous phase and distillation of the organic phase.

In step 1 the hydroxide is preferably in a quantity ranging from 5 to 15% by weight with respect the total weight of the solution, preferably said hydroxide is potassium hydroxide.

A preferred aspect is that the reaction be carried out in the presence of a metal sulfate, preferably zinc sulfate, at a concentration ranging from 1·10⁻⁵ to 5.0 moles/litre with respect to the aqueous phase alone.

According to a preferred aspect of the present invention, the selective hydrogenation process of aromatic compounds, such as benzene, or polyunsaturated compounds, is carried out in an autoclave. The temperature preferably ranges from 120 to 180°C and the pressure preferably ranges from 4 to 6 MPa. In particular, under the above-mentioned conditions, in order to maximize the yield, for example, to cyclohexene and minimize the yield, for example, to cyclohexane, the reaction times preferably range from 40 to 80 minutes.

Some illustrative and non-limiting examples are provided hereunder for a better understanding of the present invention and for its practical embodiment.

### Example 1-Synthesis of Ti (η⁶-benzene) (AlCl₄)₂ (P-B-1)

6.72 g (504 mmoles) of sublimated AlCl₃ and TiCl₄ (4.55 g, 24 mmoles) are added to a suspension of aluminium in powder form (3.95 g, 146 mmoles) in benzene (150 mL). The mixture is left at reflux temperature (120°C) for 20 hours. It is then filtered on a porous septum and the solution obtained is subsequently reduced to about 30 mL by evaporation of the solvent under vacuum. 150 mL of n-heptane are added to the residue and the mixture obtained is then placed in a refrigerator at about 4°C, for 24 hours. The purplish solid precipitated is recovered by rapid filtration of the suspension, washed with heptane and dried under vacuum.

Upon ICP elemental analysis, said solid proves to have the following metal content (weight %): Ti 10.3%, Al 12.5%, whereas the content of Cl, determined by means of potentiometric titration, proves to be equal to 62.8%.

### Example 2 - Synthesis of Zr(η⁶-benzene)₂(Al₃Cl₁₁) (P-B-2)

A suspension of aluminium in powder form (5.06 g, 187.5 mmoles) in benzene (430 mL) was treated with fresh sublimated AlCl₃ (8.60 g, 64.5 mmoles) and ZrCl₄ (7.16 g, 30.74 mmoles). The mixture was left at reflux temperature (120°C) for 24 hours. The suspension was then filtered under heat on a G3 filter, and the solution obtained was subsequently reduced to about 100 mL by evaporation of the solvent under vacuum. 150 mL of anhydrous n-heptane were added to the residue and the mixture was left under vigorous stirring for about 1 hour and then placed in a refrigerator at about 4°C, for 24 hours. The dark solid precipitated was recovered by rapid filtration of the cold suspension, washed with benzene and dried under vacuum, obtaining 10.5 g.

Upon ICP elemental analysis, said solid proved to have the following metal content (weight %): Zr 11.7%, Al 12.8%, whereas the content of Cl, determined by means of potentiometric titration, proved to be equal to 55%.

UV-Vis analysis (benzene) revealed the following three bands: at 366 nm (weak), at 416 nm (intense), at 492 nm (weak).

### Example 3 - preparation of the catalyst

40 mL of a solution of KOH at 10% are introduced into a 250 mL flask and 1 mL of Zr(OPr)₄ at 70% in i-PrOH are then rapidly added under stirring. The formation of ZrO₂ is practically immediate. It is left to digest under stirring for 15 minutes. The following products are rapidly added to the suspension, in sequence: 10 mL of a solution of FeCl₃·7H₂O at 4% by weight, left to homogenize for 15 minutes, then 10 ml of H₂O with 60 mg of Ga₂(SO₄)₃·H₂O dispersed therein, and finally 10 ml of a solution of ZnSO₄·H₂O at 0.6% by weight. The suspension is left under vigorous stirring for a further 15 minutes, subsequently centrifuged at 2000 revs for 10 minutes and the precipitate recovered and dried. The solid is transferred to a dry box in an anhydrous and oxygen-free atmosphere, and then re-dispersed in anhydrous benzene under stirring. 10 mL of a solution of the precursor P-B-1 prepared in Example 1 and the precursor P-B-2 in benzene (100 mg + 100 mg in 10 mL) are added to the dispersion thus obtained and the suspension is left under stirring for 20 hours at room temperature. The suspension is extracted from the dry box and water is added, the solid is finally centrifuged at 2000 revs for 10 minutes. The solid is washed with ether, centrifuged again and dried in an oven at 100°C. The solid is subsequently dispersed in an aqueous solution of KOH at 10% and a solution of RuCl₃ (10 mL at 3.2% as Ru) is added to this. The suspension is then brought to a temperature of 80°C, always under stirring, in 60 minutes, by means of a thermostat-regulated oil bath, and left at this temperature for 1 hour.

The stirring and heating are interrupted and the solid is left to cool and settle for 18 hours. After this period, the supernatant liquid is eliminated after centrifugation at 2000 revs for 10 minutes. The solid is dispersed in 40 ml of H₂O and transferred to a 250 ml autoclave coated with PTFE. The reactor is pressurized with 40 atm of H₂ and brought to a temperature of 150°C in an hour under stirring (5000 revs per minute), obtaining a final pressure of 50 atm. The system is left under these temperature and pressure conditions under mechanical stirring for 1 hour, then left to cool slowly and settle for 18 hours. The autoclave is depressurized and the suspension of the catalyst is transferred to a glass to which 40 mL of H₂O are added. The whole mixture is left under stirring in air for 5 hours in order to obtain passivated particles of Ru and avoid oxidation phenomena. The solid is finally separated from the liquid by centrifugation at 2000 revs for 10 minutes and dried under vacuum with a rotating pump at 3 torr for 20 hours. About 0.9 g of catalyst with 40% of Ru were obtained from this preparation, which are preserved in a drier.

### Example 4

For the hydrogenation tests of benzene, a 250 mL reactor (autoclave) was used, of the semi-continuous type, made of Hastelloy C4, equipped with a glass made of PTFE with 4 breakwaters. The stirring is guaranteed by a self-suction turbine made of Hastelloy C4 connected to a magnetic entrainment system. The thermostat regulation of the reactor is ensured by an oil bath equipped with a temperature regulation system, directly connected to a thermoresistance positioned inside the same reactor. The charging of benzene is effected by introduction from a loading autoclave thermostat-regulated at the reaction temperature.

40 mL of an aqueous solution of ZnSO₄·7H₂O at 18% and 120 mg of Ru-based catalyst, as previously prepared in example 3, are charged into the reactor. Benzene is charged into the loading autoclave and the whole system is purged of air with N₂, then with H₂ and pressurized at 30 bar of H₂. The temperature of the autoclaves is then brought to 150°C in an hour and the pressure rises to about 40 bar, the system is left under these temperature and pressure conditions for 1 hour before starting the reaction, to allow a reduction in the passivation layer of Ru and subsequently the activation of the catalyst. The pressure in the reaction autoclave is brought to 50 bar and the two autoclaves are put in communication with each other. The benzene flows rapidly into the main reactor and a stirring rate of 1500 revs per minute is then established. The pressure in the reactor is kept constant by means of a pressure regulator.

The reaction kinetics is followed by sampling of the organic phase. The sampling is effected by interrupting the stirring and the sample is analyzed via gas-chromatography.

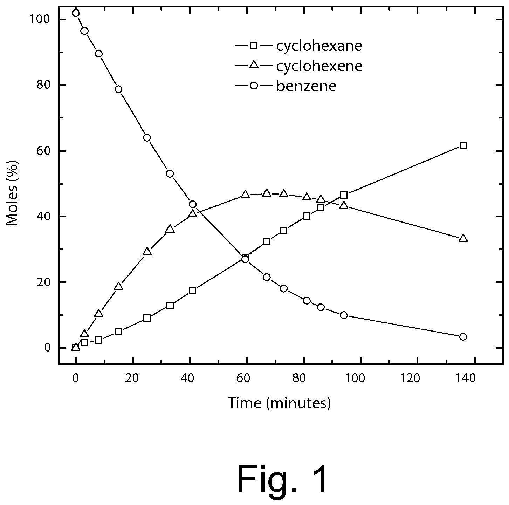

Figure 1 indicates the concentration trend against time for reagents and products in the organic phase relating to the catalyst of Example 3. Table 1 indicates the initial reaction rate (r₀), the initial selectivity % and yield %.

The initial reaction rate (r₀) is the reaction rate extrapolated at time 0 expressed in 10² mol (benzene) L⁻¹ s⁻¹ g_{cat}⁻¹.

The initial selectivity is the selectivity in moles of cyclohexene with respect to moles converted % extrapolated at time 0.

Yield refers to the moles of cyclohexene with respect to the total moles %.

### Example 5

The same procedure is effected as in Example 4, using a solution containing only the precursor P-B-1 obtained in Example 1 (100 mg of P-B-1 in 10 mL of benzene). Table 1 indicates the initial rate r₀, the initial selectivity % and the yield %.

### Example 6

The same procedure is effected as in Example 4, using a solution containing only the precursor P-B-2 obtained in Example 2 (100 mg of P-B-2 in 10 mL of benzene). Table 1 indicates the initial rate r₀, the initial selectivity % and the yield %.

### Example 7

The same procedure is effected as in Example 4, changing the composition of the solution containing the precursors of the promoters (B): P-B-1 50 mg, P-B-2 100 mg benzene 10 mL. Table 1 indicates the initial rate r₀, the initial selectivity % and the yield %.

### Example 8

The same procedure is effected as in Example 4, changing the composition of the solution containing the precursors of the promoters (B): P-B-1 100 mg, P-B-2 50 mg benzene 10 mL. Table 1 indicates the initial rate r₀, the initial selectivity % and the yield %.

### Example 9

The same procedure is effected as in Example 4, changing the composition of the solution containing the precursors of the promoters (B): P-B-1 100 mg, P-B-2 200 mg benzene 10 mL. Table 1 indicates the initial rate r₀, the initial selectivity % and the yield %.

### Example 10

The same procedure is effected as in Example 4, changing the composition of the solution containing the precursors of the promoters (B): P-B-1 200 mg, P-B-2 100 mg benzene 10 mL. Table 1 indicates the initial rate r₀, the initial selectivity % and the yield %.

### Example 11

The same procedure is effected as in Example 4, changing the composition of the solution containing the precursors of the promoters (B): P-B-1 50 mg, P-B-2 100 mg benzene 10 mL. Table 1 indicates the initial rate r₀, the initial selectivity % and the yield %.

### Example 12

The same procedure is effected as in Example 4, without using organometallic promoters.

Table 1 below indicates the results of the activity and selectivity of the catalysts based on Ru of the present invention in the hydrogenation of benzene to cyclohexene. Reaction conditions:
T = 150°C, P = 50 bar, benzene 40 ml, 40 ml of a solution of ZnSO₄ 0.6 mol L⁻¹, catalyst 120 mg.

In Table 1, the catalysts are represented by the relative components and by means of the precursors P-B-1 and P-B-2 used, even if in the catalyst, said precursors have generated the corresponding promoters:

**Table 1**

| Ex. | Catalyst | P-B-1 | P-B-2 | Initial rate r₀ | Initial select % | Yield % |
|---|---|---|---|---|---|---|
| 4 | Ru/ZrO₂/Fe/Ga/Zn/(P-B-1)/(P-B-2) | 100 | 100 | 11 | 86 | 55 |
| 5 | Ru/ZrO₂/Fe/Ga/Zn/(P-B-1) | 100 | 0 | 6.8 | 82 | 50 |
| 6 | Ru/ZrO₂/Fe/Ga/Zn/(P-B-2) | 0 | 100 | 7.6 | 84 | 52 |
| 7 | Ru/ZrO₂/Fe/Ga/Zn/(P-B-1)/0,5(P-B-2) | 100 | 50 | 8.7 | 82 | 51 |
| 8 | Ru/ZrO₂/Fe/Ga/Zn/(P-B-1)/2(P-B-2) | 100 | 200 | 9.8 | 82 | 52 |
| 9 | Ru/ZrO₂/Fe/Ga/Zn/0,5(P-B-1)/(P-B-2) | 50 | 100 | 9.1 | 83 | 52 |
| 10 | Ru/ZrO₂Fe/Ga/Zn/2(P-B-1)/(P-B-2) | 200 | 100 | 8.9 | 82 | 49 |
| 11 | Ru/ZrO₂/Fe/Ga/Zn | 0 | 0 | 7.5 | 82 | 46 |

r₀ = 10² mol L⁻¹ s⁻¹ g_{cat}⁻¹

As appears evident from the results indicated above, the catalysts prepared using compounds having formula (III) as precursors, show a much higher yield than that obtainable with the catalysts of the prior art based on Ru, containing as promoters only oxides of metals belonging to group IB, IIB and IIIA, in particular Ga and Zn.

## Claims

1. A catalyst comprising:
- a zirconia carrier (ZrO₂)
- ruthenium
- iron oxide (Fe₂O₃)
- at least one promoter (A) selected from the oxides of metals of groups IB, IIB and IIIa
- at least one promoter (B) containing V, Ti, Hf, Zr or a mixture thereof, obtained by hydrolysis of at least one precursor P-B selected from the following metal arenes:
V°(arene)₂ (I)
V(arene)₂ AlCl₄ (II)
M(η⁶-arene)ₚAl_{q}Xᵣ (III)
M(η⁶-arene)_{p'}Al_{q'}X_{r'}Rₛ (IV)
wherein:
- V represents vanadium,
- Al represents aluminium,
- M represents zirconium (Zr), hafnium (Hf), titanium (Ti) or mixtures thereof;
- arene represents a benzene, or a benzene substituted by from 1 to 6 linear or branched C₁-C₆ alkyl groups, or mixtures thereof;
- X represents a halogen atom, selected from chlorine, bromine, fluorine, iodine, preferably chlorine;
- R represents a linear or branched C₁-C₁₅ alkyl group;
- p represents a number ranging from 1 to 2;
- q is a number ranging from 2 to 6, preferably from 2 to 3;
- r is a number ranging from 8 to 20, preferably from 8 to 11;
- p' represents a number ranging from 1 to 2;
- q' is a number ranging from 2 to 6, preferably from 2 to 3;
- r' is a number ranging from 2 to 20, preferably from 6 to 9;
- s is a number ranging from 1 to 6, preferably 2.

2. The catalyst according to claim 1, containing ruthenium in a quantity ranging from 20% to 55% by weight, with respect to the weight of the final catalyst, expressed as metal element.

3. The catalyst according to claim 1, wherein the iron oxide is in a quantity ranging from 1% to 20% by weight, with respect to the weight of the catalyst.

4. The catalyst according to claim 1, wherein the promoter (A) is selected from gallium oxide (Ga₂O₃), zinc oxide (ZnO), copper oxide (CuO) and mixtures thereof.

5. The catalyst according to claim 1, wherein the promoter (A) is in a quantity ranging from 1 to 20% by weight, with respect to the metallic ruthenium.

6. The catalyst according to claim 1, wherein Ti, Zr, Hf and V are present in the final catalyst in a quantity ranging from 0.2 to 5% by weight, expressed as weight of the metal, with respect to the weight of the catalyst 1.

7. A process for preparing catalysts according to claim 1, which comprises;
- a precipitation phase in a basic aqueous solution of a zirconium alcoholate which precipitates in the form of finely subdivided ZrO₂,
- a precipitation phase of the iron oxide and promoter oxides (A) for feeding the corresponding hydrolyzable salts,
- an addition phase of one or more precursors P-B of the promoter(s) (B), wherein the precursor of the promoter (B) is selected from metal arenes having general formula (I), (II), (III), (IV) or mixtures thereof,
- an addition phase of a hydrolyzable salt of ruthenium,
- hydrothermal treatment of the suspension resulting from the previous phases.

8. The process according to claim 7, wherein, in the metal arenes having general formula (I), (II), (III), (IV), said arenes are selected from benzene, toluene, ortho-xylene, meta-xylene, para-xylene, 1,2,3-trimethylbenzene, 1,2,4-trimethylbenzene, 1,3,5-trimethylbenzene, hexamethylbenzene, or mixtures thereof.

9. The process according to claim 7 or 8 comprising:
a. adding a zirconium alcoholate in an alcohol solution, preferably a solution of the alcohol of the corresponding alcoholate, to a basic aqueous solution, preferably a solution of KOH, obtaining a precipitate of ZrO₂;
b. adding at least one hydrolyzable iron salt and a hydrolyzable salt of a metal selected from metals of group IB, IIB and IIIA;
c. separating the precipitate and drying it;
d. re-suspending the solid resulting from the drying in a solvent selected from benzene, toluene and xylenes and adding one or more precursors P-B of promoters (B) selected from metal-arenes having formula (I), (II), (III) or (IV) in a solution of the same solvent, to the resulting suspension;
e. separating the solid resulting from the previous step, re-dispersing it in an aqueous solution of a base, preferably KOH, and adding a hydrolyzable salt of Ru;
f. separating the solid obtained in the previous step, re-dispersing it in water and maintaining it in an autoclave under hydrothermal conditions;
g. separating the solid obtained in step (f).

10. The process according to claim 7 or 9, wherein the hydrothermal treatment is carried out at a temperature ranging from 120 to 180°C and a hydrogen pressure ranging from 3 to 6 MPa.

11. A selective hydrogenation process of aromatic or polyunsaturated compounds which comprises putting the aromatic or polyunsaturated compound in contact with hydrogen in the presence of the catalyst according to one or more of claims 1 to 6.

12. The process according to claim 11, comprising:
1 charging the catalyst according to one or more of claims 1 to 6 into an aqueous solution containing an alkaline hydroxide;
2 charging the aromatic or polyunsaturated compound into the aqueous solution, under stirring;
3 reacting the aromatic or polyunsaturated compound with a continuous flow of hydrogen;
4 at the end of the reaction, recovering the selectively hydrogenated compound by demixing the organic phase from the aqueous phase and distillation of the organic phase.

13. The process according to claim 11 or 12, carried out at a temperature ranging from 120 to 180°C and a pressure ranging from 4 to 6 MPa.

## Patentansprüche

1. Katalysator, umfassend:
- einen Zirkonia-Träger (ZrO₂)
- Ruthenium
- Eisenoxid (Fe₂O₃)
- mindestens einen Beschleuniger (A), ausgewählt aus den Oxiden der Metalle der Gruppen IB, IIB und IIIa
- mindestens einen Beschleuniger (B), enthaltend V, Ti, Hf, Zr oder eine Mischung davon, erhalten durch Hydrolyse von mindestens einem Vorläufer P-B, ausgewählt aus den folgenden Metallarenen:
V° (Aren)₂ (I)
V (Aren)₂ AlCl₄ (II)
M(η⁶-Aren)ₚAl_{q}Xᵣ (III)
M(η⁶-Aren)_{p'}Al_{q'}X_{r'}Rₛ (IV)
wobei:
- V steht für Vanadium,
- Al steht für Aluminium,
- M steht für Zirkonium (Zr), Hafnium (Hf), Titan (Ti) oder Mischungen davon;
- Aren steht für ein Benzen oder ein Benzen, substituiert durch von 1 bis 6 linearen oder verzweigten C₁-C₆-Alkylgruppen oder Mischungen davon;
- X steht für ein Halogenatom, ausgewählt aus Chlor, Brom, Fluor, Iod, vorzugsweise Chlor;
- R steht für eine lineare oder verzweigte C₁-C₁₅-Alkylgruppe;
- p steht für eine Zahl in einem Bereich von 1 bis 2;
- q ist eine Zahl in einem Bereich von 2 bis 6, vorzugsweise von 2 bis 3;
- r ist eine Zahl in einem Bereich von 8 bis 20, vorzugsweise von 8 bis 11;
- p' steht für eine Zahl in einem Bereich von 1 bis 2;
- q' ist eine Zahl in einem Bereich von 2 bis 6, vorzugsweise von 2 bis 3;
- r' ist eine Zahl in einem Bereich von 2 bis 20, vorzugsweise von 6 bis 9;
- s ist eine Zahl in einem Bereich von 1 bis 6, vorzugsweise 2.

2. Katalysator nach Anspruch 1, enthaltend Ruthenium in einer Menge in einem Bereich von 20 bis 55 Gew.-%, bezogen auf das Gewicht des finalen Katalysatoren, ausgedrückt als Metallelement.

3. Katalysator nach Anspruch 1, wobei das Eisenoxid in einer Menge in einem Bereich von 1 bis 20 Gew.-% ist, bezogen auf das Gewicht des Katalysatoren.

4. Katalysator nach Anspruch 1, wobei der Beschleuniger (A) ausgewählt ist aus Galliumoxid (Ga₂O₃), Zinkoxid (ZnO), Kupferoxid (CuO) und Mischungen davon.

5. Katalysator nach Anspruch 1, wobei der Beschleuniger (A) in einer Menge in einem Bereich von 1 bis 20 Gew.% ist, bezogen auf das metallische Ruthenium.

6. Katalysator nach Anspruch 1, wobei Ti, Zr, Hf und V in dem finalen Katalysator in einer Menge in einem Bereich von 0,2 bis 5 Gew.-% vorliegen, ausgedrückt als Gewicht des Metalls, bezogen auf das Gewicht des Katalysatoren 1.

7. Verfahren zur Herstellung von Katalysatoren nach Anspruch 1, welches umfasst:
- eine Ausfällungsphase in einer basischen wässrigen Lösung eines Zirkoniumalkoholats, das in Form von fein zerteiltem ZrO₂ ausfällt,
- eine Ausfällungsphase des Eisenoxids und Beschleunigeroxide (A) zur Einspeisung der entsprechenden hydrolysierbaren Salze,
- eine Zugabephase von einem oder mehreren Vorläufern P-B des Beschleunigers/der Beschleuniger (B), wobei der Vorläufer des Beschleunigers (B) ausgewählt ist aus Metallarenen mit der allgemeinen Formel (I), (II), (III), (IV) oder Mischungen davon,
- eine Zugabephase eines hydrolysierbaren Salzes von Ruthenium,
- hydrothermale Behandlung der sich aus den vorhergehenden Phasen ergebenden Suspension.

8. Verfahren nach Anspruch 7, wobei, bei den Metallarenen mit der allgemeinen Formel (I), (II), (III), (IV), diese Arenen ausgewählt sind aus Benzen, Toluen, ortho-Xylen, meta-Xylen, para-Xylen, 1,2,3-Trimethylbenzen, 1,2,4-Trimethylbenzen, 1,3,5-Trimethylbenzen, Hexamethylbenzen oder Mischungen davon.

9. Verfahren nach Anspruch 7 oder 8, umfassend:
a. Zugabe eines Zirkoniumalkoholats in eine alkoholische Lösung, vorzugsweise eine Lösung des Alkohols des entsprechenden Alkoholats, zu einer basischen wässrigen Lösung, vorzugsweise eine Lösung von KOH, wobei eine Ausfällung von ZrO₂ erhalten wird;
b. Zugabe mindestens eines hydrolysierbaren Eisensalzes und eines hydrolysierbaren Salzen eines Metalls, ausgewählt aus Metallen der Gruppe IB, IIB und IIIA;
c. Abtrennung der Ausfällung und Trocknung derselben;
d. Resuspendierung des nach der Trocknung erhaltenen Feststoffs in einem Lösemittel, ausgewählt aus Benzen, Toluen und Xylenen, und Zugabe von einem oder mehreren Vorläufern P-B der Beschleuniger (B), ausgewählt aus Metallarenen mit der Formel (I), (II), (III) oder (IV), in einer Lösung des gleichen Lösemittels, zu der erhaltenen Suspension;
e. Abtrennung des aus dem vorhergehenden Schritt erhaltenen Feststoffs, Redispergierung desselben in einer wässrigen Lösung einer Base, vorzugsweise KOH, und Zugabe eines hydrolysierbaren Salzes von Ru;
f. Abtrennung des in dem vorhergehenden Schritt erhaltenen Feststoffs, Redispergierung desselben in Wasser und Halten desselben in einem Autoklaven unter hydrothermalen Bedingungen;
g. Abtrennung des in Schritt (f) erhaltenen Feststoffs.

10. Verfahren nach Anspruch 7 oder 9, wobei die hydrothermale Behandlung durchgeführt wird bei einer Temperatur in einem Bereich von 120 bis 180°C und einem Wasserstoffdruck in einem Bereich von 3 bis 6 MPa.

11. Selektives Hydrierungsverfahren von aromatischen und polyungesättigten Verbindungen, welches umfasst das In-Kontaktbringen der aromatischen oder polyungesättigten Verbindung mit Wasserstoff in Gegenwart eines Katalysatoren nach irgendeinem der Ansprüche 1 bis 6.

12. Verfahren nach Anspruch 11, umfassend:
1 Einbringen des Katalysatoren nach irgendeinem der Ansprüche 1 bis 6 in eine ein alkalisches Hydroxid enthaltende wässrige Lösung;
2 Einbringen der aromatischen oder polyungesättigten Verbindung in die wässrige Lösung unter Rühren;
3 Umsetzen der aromatischen oder polyungesättigten Verbindung mit einem kontinuierlichen Fluss Wasserstoff;
4 am Ende der Umsetzung, Gewinnen der selektiv hydrierten Verbindung durch Entmischen der organischen Phase von der wässrigen Phase und Destillierung der organischen Phase.

13. Verfahren nach Anspruch 11 oder 12, ausgeführt bei einer Temperatur in einem Bereich von 120 bis 180°C und einem Druck in einem Bereich von 4 bis 6 MPa.

## Revendications

1. Catalyseur comprenant :
- un support en zircone (ZrO₂),
- du ruthénium,
- de l'oxyde de fer (Fe₂O₃),
- au moins un promoteur (A) choisi parmi les oxydes des métaux des groupes I-B, II-B et III-A,
- au moins un promoteur (B) contenant du vanadium, du titane, du hafnium ou du zirconium, ou un mélange de ces éléments, obtenu par hydrolyse d'au moins un précurseur (P-B) choisi parmi les composés métal-arène suivants :
V⁰(arène)₂ (I)
V(arène)₂AlCl₄ (II)
M(η⁶-arène)ₚAl_{q}Xᵣ (III)
M(η⁶-arène)_{p'}Al_{q'}X_{r'}Rₛ (IV)
formules dans lesquelles
- V représente un atome de vanadium,
- Al représente un atome d'aluminium,
- M représente un atome de zirconium (Zr), de hafnium (Hf) ou de titane (Ti), ou une combinaison de tels atomes,
- « arène » représente le benzène ou un dérivé du benzène, porteur, en tant que substituant(s), de 1 à 6 groupe(s) alkyle en C₁-C₆, linéaire(s) ou ramifié(s), ou un mélange de tels composés,
- X représente un atome d'halogène choisi parmi chlore, brome, fluor et iode, et de préférence un atome de chlore,
- R représente un groupe alkyle en C₁-C₁₅, linéaire ou ramifié,
- l'indice p est un nombre valant de 1 à 2,
- l'indice q est un nombre valant de 2 à 6, de préférence de 2 à 3,
- l'indice r est un nombre valant de 8 à 20, de préférence de 8 à 11,
- l'indice p' est un nombre valant de 1 à 2,
- l'indice q' est un nombre valant de 2 à 6, de préférence de 2 à 3,
- l'indice r' est un nombre valant de 2 à 20, de préférence de 6 à 9,
- et l'indice s est un nombre valant de 1 à 6, de préférence 2.

2. Catalyseur conforme à la revendication 1, qui contient du ruthénium en une proportion valant de 20 à 55 %, en poids rapporté au poids du catalyseur final, exprimé en élément métal.

3. Catalyseur conforme à la revendication 1, dans lequel l'oxyde de fer se trouve présent en une proportion valant de 1 à 20 %, en poids rapporté au poids du catalyseur.

4. Catalyseur conforme à la revendication 1, dans lequel le promoteur (A) est choisi parmi de l'oxyde de gallium (Ga₂O₃), de l'oxyde de zinc (ZnO), de l'oxyde de cuivre (CuO) et des mélanges de ces oxydes.

5. Catalyseur conforme à la revendication 1, dans lequel le promoteur (A) se trouve présent en une proportion valant de 1 à 20 %, en poids, par rapport au ruthénium métal.

6. Catalyseur conforme à la revendication 1, dans lequel Ti, Zr, Hf et V se trouvent présents dans le catalyseur final en une proportion valant de 0,2 à 5 % en poids, exprimée en poids de métal rapporté au poids du catalyseur 1.

7. Procédé de préparation de catalyseurs conformes à la revendication 1, lequel procédé comporte :
- une étape où l'on fait précipiter de la zircone ZrO₂ sous forme finement divisée, dans une solution aqueuse basique, à partir d'un alcoolate de zirconium,
- une étape où l'on fait précipiter de l'oxyde de fer et des oxydes promoteurs (A), par addition des sels hydrolysables correspondants,
- une étape où l'on ajoute un ou plusieurs précurseur(s) P-B de promoteur B, lequel ou lesquels précurseur(s) de promoteur B sont choisi(s) parmi les composés métal-arène de formules générales (I), (II), (III) et (IV) et leurs mélanges,
- une étape où l'on ajoute un sel hydrolysable du ruthénium,
- et un traitement hydrothermique de la suspension qui résulte des étapes précédentes.

8. Procédé conforme à la revendication 7, dans lequel, dans les composés métal-arène de formules générales (I), (II), (III) et (IV), les arènes sont choisis parmi les benzène, toluène, ortho-xylène, métaxylène, para-xylène, 1,2,3-triméthyl-benzène, 1,2,4-triméthyl-benzène, 1,3,5-triméthyl-benzène, hexaméthyl-benzène et leurs mélanges.

9. Procédé conforme à la revendication 7 ou 8, qui comporte les opérations suivantes :
a) ajouter un alcoolate de zirconium en solution dans un alcool, de préférence dans l'alcool constituant dudit alcoolate, à une solution aqueuse basique, de préférence une solution de potasse (KOH), ce qui donne un précipité de zircone (ZrO₂),
b) ajouter au moins un sel de fer hydrolysable et un sel hydrolysable d'un métal choisi parmi les métaux des groupes I-B, II-B et III-A,
c) séparer le précipité et le faire sécher,
d) remettre le solide issu de ce séchage en suspension dans un solvant choisi parmi les benzène, toluène et xylènes, et ajouter à la suspension ainsi obtenue un ou plusieurs précurseur(s) P-B de promoteur B, choisi(s) parmi les composés métal-arène de formules générales (I), (II), (III) et (IV), en solution dans le même solvant,
e) séparer le solide obtenu à l'issue de l'étape précédente, le disperser de nouveau dans une solution aqueuse d'une base, de préférence de potasse (KOH), et ajouter un sel hydrolysable du ruthénium,
f) séparer le solide obtenu à l'issue de l'étape précédente, le disperser de nouveau dans de l'eau, et le maintenir dans un autoclave, dans des conditions de traitement hydrothermique,
g) et séparer le solide obtenu à l'issue de l'étape (f).

10. Procédé conforme à la revendication 7 ou 9, dans lequel le traitement hydrothermique est effectué à une température valant de 120 à 180 °C et sous une pression d'hydrogène valant de 3 à 6 MPa.

11. Procédé d'hydrogénation sélective de composés aromatiques ou polyinsaturés, qui comporte le fait de mettre un composé aromatique ou polyinsaturé en contact avec de l'hydrogène, en présence d'un catalyseur conforme à l'une ou plusieurs des revendications 1 à 6.

12. Procédé conforme à la revendication 11, qui comporte les étapes suivantes :
1) introduire un catalyseur, conforme à l'une ou plusieurs des revendications 1 à 6, dans une solution aqueuse contenant un hydroxyde de métal alcalin,
2) introduire un composé aromatique ou polyinsaturé dans la solution aqueuse, tout en agitant le tout,
3) faire réagir le composé aromatique ou polyinsaturé avec de l'hydrogène passant en courant continu,
4) et à la fin de la réaction, récupérer le composé sélectivement hydrogéné, par démixtion de la phase organique d'avec la phase aqueuse et distillation de la phase organique.

13. Procédé conforme à la revendication 11 ou 12, mis en œuvre à une température valant de 120 à 180 °C et sous une pression valant de 4 à 6 MPa.
